# EUROPEAN PATENT APPLICATION

(11) **EP 2 093 234 A1**
(43) Date of publication of application: **26.08.2009**
(21) Application number: 08002327.8
(22) Date of filing: 08.02.2008
(51) Int. Cl.: C07K 5/04, C07K 7/06, A23L 1/305

(54) **Oligopeptides for use as taste modulators**

(71) Applicant: Nutrinova Nutrition Specialties & Food Ingredients GmbH, 65926 Frankfurt am Main (DE)
(72) Inventor: Krohn, Michael, Dr., 64653 Lorsch (DE); Zinke, Holger, Dr., 64646 Heppenheim/Sonderbach (DE)

(57) **Abstract**

The invention relates to the use of one or more oligopeptide/s, preferably a tripeptide such as aladapcin, as a taste modulator and/or sweetness enhancer for comestible compositions preferably those containing at least one natural or artificial sweetener. The comestible compositions include food, beverages, medicinal products and cosmetics and contain preferably mono-, di- or oligosaccharides as sweeteners. The invention further relates to said comestible compositions containing an oligopeptide as taste modulator.

## Description

The present invention relates to the use of oligopeptides as taste modulators for comestible compositions containing at least one sweetener. Furthermore, this invention relates to a method for the modulation of taste and/or aftertaste of said comestible compositions as well as to such compositions containing at least one oligopeptide as taste modulator.

In this specification, a number of documents are cited, the entire disclosures of these references (including i. a. scientific articles, patents and patent applications) are hereby incorporated herein by reference for the purpose of describing at least in part the knowledge of those of ordinary skill in the art and for the purpose of disclosing e.g. compounds, structures (such as T2Rs and T1Rs mammalian taste receptor proteins) and methods for e.g. expressing those receptors in cell lines and using the resulting cell lines for screening compounds with regard to their taste modulating activity.

There has been significant recent progress in identifying useful derivatives of natural flavouring agents, such as for example sweeteners that are derivatives of natural saccharide sweeteners, such as for example erythritol, isomalt, lactitol, mannitol, sorbitol, xylitol. There has also been recent progress in identifying natural terpenoids, flavonoids, or proteins as potential sweeteners. See, for example, a recent article entitled "Noncariogenic Intense Natural Sweeteners" by Kinghorn et al. (Med. Res Rev (1998)18(5):347-360), which discussed recently discovered natural materials that are much more intensely sweet than common natural sweeteners such as sucrose, fructose, glucose, and the like. Similarly, there has been recent progress in identifying and commercializing new artificial sweeteners, such as aspartame, saccharin, acesulfame-K, cyclamate, sucralose, and alitame, etc.; see an article by Ager et al. (Angew. Chem. Int. Ed. (1998) 37,1802-1817).

In recent years substantial progress has been made in biotechnology in general and in better understanding the underlying biological and biochemical phenomena of taste perception. For example, taste receptor proteins have been recently identified in mammals that are involved in taste perception. Particularly, two different families of G protein coupled receptors believed to be involved in taste perception, T2Rs and T1Rs, have been identified. (See, e. g., Nelson et al., Cell (2001) 106(3):381-390; Adler et al., Cell (2000) 100(6):693-702; Chandrashekar et al., Cell (2000) 100:703-711; Matsunami et al., Number (2000) 404:601-604; Li et al., Proc Natl Acad Sci USA (2002) 99:4962-4966; Montmayeur et al., Nature Neuroscience (2001) 4(S):492-498; U.S. Patent 6,462,148; and PCT publications WO 02/06254, WO 00/63166 art, WO 02/064631, and WO 03/001876, and U.S. Patent Publication US 2003-0232407 A1.

Whereas the T2R family includes over 25 genes that are involved in bitter taste perception, the T1 R family responsible for sweet perception only includes three members, T1R1, T1 R2 and T1 R3 (cf. Li et al., Proc. Natl. Acad. Sci. USA (2002) 99, 4962-4966). Recently, it was disclosed in WO 02/064631 and WO 03/001876 that certain T1 R members, when co-expressed in suitable mammalian cell lines, assemble to form functional taste receptors. It was found that co-expression of T1 R2 and T1 R3 in a suitable host cell results in a functional T1 R2/T1 R3 "sweet" taste receptor that responds to different taste stimuli including naturally occurring and artificial sweeteners (cf. Li et al., cited hereinabove).

Food, beverages, pleasing products, sweetenings, pet foods, medicinal products or cosmetics often do have a high content of sweeteners, which is generally regarded as undesirable in terms of sweetener related disease development. Here especially diseases like obesity, diabetes, cardio vascular diseases and others are prone mainly to high caloric sweeteners. There is good evidence that increased uptake of high caloric sweeteners, e. g. mono-, di- and oligosaccharides especially sucrose, is linked to higher levels of plasma triacylglycerides which is an accepted risk factor for cardiovascular disease. Likewise increased sugar uptake can be linked to a physical status which promotes diabetes, obesity or other diseases. In the food and beverage industry it is state of the art to replace those troubling sugars like glucose, saccarose, trehalose and others with fructose.

The global sweetener market is currently at a scale of 170 million tons per year of sugar-equivalent (units of measurement to compare amounts of different sweeteners, taking into account their different sweetness potency) in 2005. This market comprises caloric sweeteners, high-intensity sweeteners and polyols. The most important caloric sweetener is refined sugar or sucrose; other caloric sweeteners are high fructose corn syrup, glucose and dextrose. High-intensity sweeteners are products that provide the same sweetness as sugar with less material and therefore fewer calories. They provide 35 to 10,000 times the sweetness of sugar. They are also known as low-caloric or dietetic sweeteners or, if they do not include any calories, non-caloric sweeteners. Apart from acesulfame-K, other important high-intensity sweeteners are saccharin, aspartame, cyclamate, stevioside and sucralose. Lastly, polyols are sugar alcohols, which provide the bulk and texture of sugar but can be labelled as having fewer calories than sugar.

For instance the use of high fructose corn syrup (HFCS) as sweeteners in baked goods (HFCS 90), soft drinks (HFCS 55), sports drinks (HFCS 42) or in breads, cereals condiments etc. is commonly accepted. HFCS refers to a group of corn syrups which are enzymatically processed in order to increase their fructose content and are then mixed with pure corn syrup (100% glucose) to reach their final form. The typical types of HFCS are HFCS 90 (approximately 90% fructose and 10% glucose); HFCS 55 (approximately 55% fructose and 45% glucose); and HFCS 42 (approximately 42% fructose and 58% glucose).

However, conclusions from recent studies can be drawn that the effects of fructose compared to sucrose on blood glucose, insulin, leptin, and ghrelin levels exhibit no significant differences. Taken together there is little or no evidence for the hypothesis that HFCS is different from sucrose in its effects on appetite or on metabolic processes involved in fat storage.

Another strategy to reduce caloric sweeteners, in e. g. packaged food, is the use of non- or low-caloric artificial sweeteners like acesulfame-K, saccharin, cyclamate, aspartame, thaumatin or neohesperidin DC, sucralose, neotame or steriol glycosides. Here two aspects are of major impact. Firstly these compounds compared to saccharides have a distinct aftertaste and secondly there is a permanent discussion whether or not these sweeteners are carcinogenic.

It is therefore desirable and an object of the present invention to find compounds with properties to modulate sweet taste, or to enhance the sweet taste evoked by a sweetener known in the art either by being sweet on their own, or being a moderate to weak sweetener on its own with enhancing attributes for one or more sweetener(s) known in the art, or most preferably being an enhancer with no sweetening attributes on its own but the ability to enhance one or more sweeteners known in the art which are used in comestible compositions.

In the art several proposals have been made with regard to compounds showing taste modulating activity. WO 2006/138512 A2 discloses bis-aromatic amides and their uses as sweet flavour modifiers, tastants and taste enhancers. US 7,175,872 B2 relates to pyridinium-betain compounds for use as taste modulators.

Nevertheless, there remains in the art a need for new and improved taste modulators of flavouring agents especially for compounds having no or only very little sweetener potential for the reasons outlined above.

The present invention solves these problems.

One aspect of the invention is the use of an oligopeptide or of a mixture of different oligopeptides as a taste modulator in comestible compositions containing one or more natural or artificial sweeteners examples of which are described above. Another aspect of the present invention is a method for the modulation of taste (including aftertaste) of the above mentioned comestible compositions comprising combining such compositions with a taste modulating amount of an oligopeptide or mixture of oligopeptides. And still another aspect of the invention relates to a comestible composition containing one or more natural or artificial sweeteners and a taste modulating amount of an oligopeptide or a mixture of oligopeptides.

For the purpose of the present invention the following terms shall have the meanings described below:
Comestible composition is to be understood in its broadest sense including but not limited to food, beverages, soft drinks, pleasing products, sweets, sweetenings, cosmetics such as for example mouthwash, animal food such as pet foods, and pharmaceuticals or medicinal products.

Taste modulator or taste modulation refers to a compound that modulates the taste (including aftertaste) of a comestible composition containing one or more natural or artificial sweeteners. A taste modulator may modulate, enhance potentiate or induce the taste impression in an animal or a human and preferably in the sense of enhanced sweet taste.

Natural and artificial sweeteners are those sweetening agents known and/or used in the art with respect to comestible compositions; examples of which are given in the preceding paragraphs.

A taste modulating amount refers to an amount of a compound or compounds capable of modulating the taste of sweetener containing comestible compositions. The concentration of a taste modulator needed to modulate or improve the taste of the comestible composition will of course depend on many variables, including the specific type of comestible composition and its various other ingredients, especially the presence of other natural and/or artificial sweeteners and the concentrations thereof, the natural genetic variability and individual preferences and health conditions of various human beings tasting the compositions, and the subjective effect of the particular compound on the taste of such sweet compounds.

Thus, it is not possible to specify an exact "effective amount". However, an appropriate effective amount can be determined by one of ordinary skill in the art using only routine experimentation (see e.g. Ex. 9 of US 7,175,872 and Ex. 53 of WO 2006/138512 A2).

The oligopeptides which can be used in the present invention are oligopeptides of the formula (I) 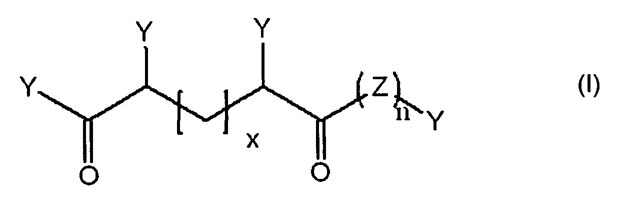 wherein
- Z: denotes the peptidic condensation product of alanine, arginine, asparagine, aspartic acid, cysteine, glutamic acid, glutamine, histidine, isoleucine, leucine, lysine, methionine, phenylalanine, proline, serine, threonine, tryptophan, tyrosine or valine wherein any of these amino acids is a β-D- or β-L-amino acid and wherein for n being >1 the Zs are identical or different,
- n: is an integer from 1 to 5
- x: is an integer from 1 to 5 and
- Y: denotes -H, -OH, -NH₂, -O-Alkyl, -O-(CO)-Alkyl, -NH-Alkyl, -N(Alkyl)₂, -NH- (CO)-Alkyl or -N((CO)-Alkyl)₂ wherein "Alkyl" is a linear or branched C₁ to C₅ alkyl rest and wherein two or more Ys are identical or different.

All such derivatives can be obtained by methods well known in the synthesis of oligopeptides.

Preferred oligopeptides of formula (I) for the use according to the present invention are those, wherein the amino acids are D-amino acids, specifically β-D-amino acids. Even more preferred is the use of oligopeptides of formula (I) wherein Z is β-D-alanine, n is 2 and x is 3 and Y is NH₂ or OH.

Most preferred is a tripeptide represented by formula (II)

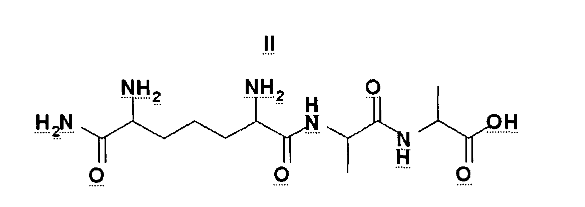

Its generic name is Aladapcin (IUPAC: 2-[2-[(2,6-diamino-6-carbamoyl-hexanoyl)-amino]propanoylamino]propanoic acid; MW: 331.368 | MF: C13H25N5O5 | XLogP: - 4.8. It was first described by (Shiraishi et al., 1990, J. Antibiot (Tokyo) 43, 634-8); and isolated from bacteria strain *Nocardia* sp. and is characterized by the above formula II).

The preferred taste modulator oligopeptides are water soluble. Thus, the preferred oligopeptides are those which contain amino acids which render the resulting oligopeptide water soluble, such as serine, lysine, or other polar amino acids.

The oligopeptides according to the invention also include derivatives. The preferred derivatives of aladapcin are those in which one or both of the D-alanines in formula I are replaced by any other D-amino acids such as D-arginine, D-asparagine, D-aspartic acid, D-cysteine, D-glutamic acid, D-glutamine, D-histidine, D-isoleucine, D-leucine, D-lysine, D-methionine, D-phenylalanine, D-proline, D-serine, D-threonine, D-tryptophan, D-tyrosine, D-valine or glycine or the corresponding L-amino acids. Thus, Z in formula I above may be any of these amino acids.

The comestible compositions to which the taste modulating oligopeptides according to the present invention are added are preferably compositions containing one or more mono-, di- or oligosaccharides as sweeteners, and most preferred are compositions containing high fructose corn syrup or high fructose syrup blends as sweeteners. Among confectionaries, cereals, ice cream, beverages, yoghurts, desserts, spreads and bakery products, nutricosmetics and medicinal compositions, preferably carbohydrated alcoholic and non-alcoholic beverages like carbonated and non-carbonated a) soft drinks, b) full calorie soft drinks, c) sport and energy drinks, d) juice drinks, e) ready-to-drink teas and other instant soft drinks, are comestible compositions of special interest for the purpose of the present invention. Most preferably are those numerous foods in which the liquid sweetener HFCS, which also constitutes a major source of dietary fructose, has become a favourite substitute for sucrose e. g. in soft drinks and many other sweetened beverages as well as in carbonate beverages, baked goods, canned fruits, jams and jellies, and dairy products.

The comestible compositions containing mono-, di- or oligosaccharides as sweeteners and an oligopeptide according to the present invention exhibit a taste quality identical or at least close to the taste of the said saccharides themselves, and especially a significantly enhanced sweetness.

The oligopeptides according to the invention and especially those of the aladapcin type significantly multiply or enhance the sweetness of known natural and/or artificial sweeteners, even when used at low concentrations, so that less of the known caloric sweeteners are required in a comestible composition, while the perceived taste of the natural sweeteners is maintained or amplified. This is of very high utility and value in view of the rapidly increasing incidence of undesirable human weight gain and/or associated diseases such as diabetes, atherosclerosis, etc.

The amount of taste modulator in the inventive comestible compositions is dependent on the concentration of the natural or artificial sweeteners contained therein as well as on the presence of further auxiliary substances such as carbon dioxide, flavors (e. g. spices, natural extracts or oils), colors, acidulants (e. g. phosphoric acid and citric acid), preservatives, potassium, sodium as to mention some of the auxiliaries. The amount desired may generally be between 0.01 mg and 10 g oligopeptide / kg of the entire finished comestible composition. The amount is in particular between 0.01 mg and 1 g oligopeptide / kg, preferably between 0.1 mg and 500 mg oligopeptide / kg, and especially between 0.1 mg and 50 mg oligopeptide / kg of the finished comestible composition (= ppm by weight).

The oligopeptides of the invention preferably have sufficient solubility in water and/or polar organic substances, and mixtures thereof, for formulation at the desired concentration ranges by simply dissolving them in the appropriate liquids. Concentration compositions comprising solid but water soluble substances such as sugars or polysaccharides, and the oligopeptides described herein can be prepared by dissolving or dispersing the oligopeptide and soluble carrier in water or polar solvents, then drying the resulting liquid, via well know processes such as spray drying.

The solubility of the oligopeptides of the invention may, however, be limited in less polar or apolar liquid carriers, such as oils or fats. In such embodiments it can be desirable to prepare a very fine dispersion or emulsion of the solid oligopeptide in the carrier, by grinding, milling or homogenizing a physical mixture of the oligopeptide and the liquid carrier. The oligopeptides can therefore in some cases be formulated as sweetener concentrate compositions comprising dispersions of solid microparticles of the oligopeptide in the precursor substances. For example, some of the oligopeptides of the invention can have limited solubility in non-polar substances such as edible fats or oils, and therefore can be formulated as sweetener concentrate compositions by milling or grinding the solid oligopeptide to microparticle size and mixing with the edible fat or oil, or by homogenizing a dispersion of the solid oligopeptide and the edible fat or oil, or a comestibly acceptable analog thereof, such as the Neobee^{™} triglyceride ester based oils sold by Stephan Corporation of Northfield Illionis, U.S.A.

It is also possible to prepare solids coated, frosted, or glazed with the well dispersed compounds of the invention by dissolving the oligopeptide in water or a polar solvent, then spraying the solid carrier or composition onto the solid comestible carrier or substrate.

By means of the methods described above, many well known and valuable comestible compositions that currently contain sugar and/or equivalent saccharide sweeteners can be reformulated to comprise one or more of the oligopeptides described herein, with a concomitant ability to reduce the concentration of the sugar and/or equivalent saccharide sweeteners significantly, e.g. by about 10% up to as much as 30 to 50%, with a corresponding drop in the caloric content of the comestible compositions.

The above described concentrate compositions are then employed in well known methods to prepare the desired comestible compositions of the invention.

Thus, the present invention encompasses different aspects all belonging to the same inventive concept:
a) the use of the oligopeptides of the invention as taste modulators for comestible compositons containing at least one known natural or artificial sweetener,
b) a method for the modulation of taste (including aftertaste) of said comestible compositions by adding one or more oligopeptides of the invention to such compositions,
c) a method for reducing the concentration of caloric sweeteners in said comestible compositions by adding one or more oligopeptides of the invention to said compositions, and
d) comestible compositions containing at least one known natural or artificial sweetener and at least one oligopeptide according to the invention.

### Examples

Further characteristics of the invention result from the following examples. In this context single characteristics of this invention alone or in combination can be realized. The following examples are provided to illustrate preferred embodiments and are intended to be illustrative and not limitative of the scope of the invention.

### Experimental materials and methods

### Cell culture

Transient transfection/ selection of stable HEK293 cells - Transient and stable transfections can be performed with lipid complexes like calcium phosphate precipitation, Lipofectamine/PLUS reagent (Invitrogen), Lipofectamine 2000 (Invitrogen) or MIRUS TransIT293 (Mirus Bio Corporation) according to the manuals. Electroporation can also be a method of choice for stable transfection of eukaryotic cells.

The cells are seeded in 6-well plates at a density of 4x10⁵ cells/well. HEK293 cells are transfected with linearised plasmids for stable expression of the genes of interest. After 24 hours, the selection with selecting reagents like zeocin, hygromycin, neomycin or blasticidin starts. About 50 µl to 300 µl trypsinized transfected cells from a 6-well are seeded in a 100 mm dish and the necessary antibiotic is added in an appropiate concentration. Cells are cultivated until clones are visible on the 100 mm cell culture plate. These clones are selected for further cultivation and calcium imaging. It takes about four to eight weeks to select cell clones which stably express the genes of interest.

### Calcium imaging

Fluo-4 AM assay with stable HEK293 cells - Stable cells are maintained in DMEM high-glucose medium (Invitrogen) supplemented with 10 % fetal bovine serum (Biochrom) and 4 mM L-glutamine (Invitrogen). Cells for calcium imaging are maintained in DMEM low-glucose medium supplemented with 10 % FBS and 1x Glutamax-1 (Invitrogen) for 48 hours before seeding. These stable cells are trypsinized after 48 hours (either with Trypsin-EDTA, Accutase or TrypLE) and seeded onto poly-D-lysine coated 96-well assay plates (Corning) at a density of 45,000 cells/well in DMEM low-glucose medium supplemented with 10 % FBS and 1x Glutamax-1.

After 24 hours, the cells were loaded in 100 µl medium with additional 100 µl of 4 µM Fluo-4 (calcium sensing dye, 2 µM end concentration; Molecular Probes) in Krebs-HEPES (KH)-buffer for 1 hour. The loading reagent is then replaced by 80 µl KH-buffer per well. The Krebs-HEPES-buffer (KH-buffer) is a physiological saline solution including 1.2 mM CaCl₂, 4.2 mM NaHCO₃ and 10 mM HEPES.

The dye-loaded stable cells in plates were placed into a fluorescence microtiter plate reader to monitor fluorescence (excitation 488 nm, emission 520 nm) change after the addition of 20 µl KH-buffer supplemented with 5x tastants. For each trace, tastant was added 11.5 seconds after the start of the scan and mixed two times with the buffer, scanning continued for an additional 32 seconds, and data were collected every second.

Data analysis / Data recording - Calcium mobilization was quantified as the change of peak fluorescence ()F) over the baseline level (F). Data were expressed as the mean S.E. of the )F/F value of replicated independent samples. The analysis was done with the software of the microtiter plate reader.

### Example 1

### Detection of aladapcin sweet enhancer activity in recombinant human taste receptor dependent T1 R2/T1 R3 dependent cell based assay

In wild type taste cells - e. g. in the human taste bud - signal transduction is presumably transduced by the G-proteins gustducin and/or by G-Proteins of the Galpha-i type. Encountering sweet ligands the heterodimeric human taste receptor T1 R2/T1 R3 reacts with induction of second messenger molecules; either induction of the cAMP level in response to most sugars or induction of the calcium level in response to most artificial sweeteners. (Margolskee, 2002; J. Biol Chem. 277, 1-4)

To analyze the function and activity of aladapcin the heterodimeric T1 R2/T1 R3 sweet taste receptor has been utilized in a calcium dependent cell based assay. T1 R type taste receptors have been transfected with the multicistronic plasmid vector pTrix-Eb-R2R3 in a HEK293 cell line stably expressing the promiscuous mouse G-alpha-15 G-protein.

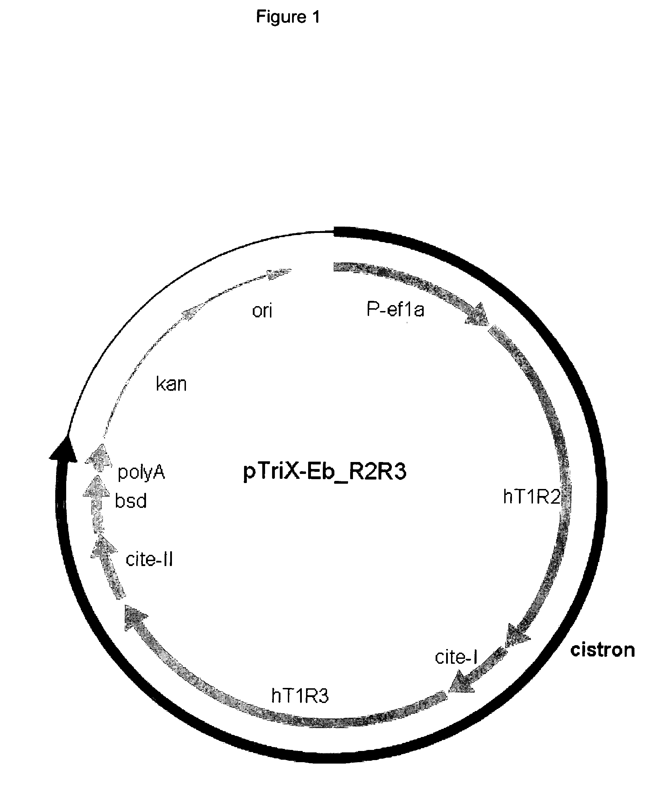

For the generation of stable cell lines a multicistronic expression unit using human taste receptor sequences have been used. As shown in Figure 1 the tricistronic expression unit of the expression vector pTrix-Eb-R2R3 is under the control of the human elongation factor 1 alpha promoter. Using standard cloning techniques the cDNA for the receptors ht1R2 and ht1R3 and the cDNA for the blasticidin S deaminase gene have been cloned. To enable the translation initiation of each gene of this tricistronic unit two EMC-virus derived internal ribosomal entry sites (IRES - also termed Cap-independent translation enhancer (CITE)) have been inserted. (Jackson et al., Trends Biochem Sci (1990) 15, 477-83; Jang et al., J Virol (1988) 62, 2636-43.)

The tricistronic expression unit is terminated by a simian virus 40 polyadenylation signal sequence. This composition permits the simultaneous expression of all three genes under the control of only one promoter. In contrast to monocistronic transcription units, which integrate independently from each other into different chromosomal locations during the process of stable cell line development, the tricistronic transcription unit integrates all containing genes in one and the same chromosomal locus. Due to the alignment of the genes, the blasticidin S deaminase gene is only transcribed in case a full length transcription takes place. Moreover the polarity of multicistronic transcription units (Moser, S. et al., Biotechnol Prog (2000) 16, 724-35) leads probably to a balanced stoichiometry of the receptor genes and their expression rates in the range of 1:0.7 up to 1:1 for the first two positions whereas the blasticidin S deaminase gene compared to the receptor genes in the third position is expressed to a lesser extend. Assuming that for the functional heterodimeric receptor ht1R2/ht1R3 a 1:1 stoichiometry is needed the lesser polarity effects for the receptor genes promote the desired stoichiometry whereas the reduced expression of the deaminase promotes an integration locus with enhanced transcriptional activity. Generation of stable T1R2/T1 R3 expressing cells have been performed by culturing the transfected cells in the presence of blasticidine.

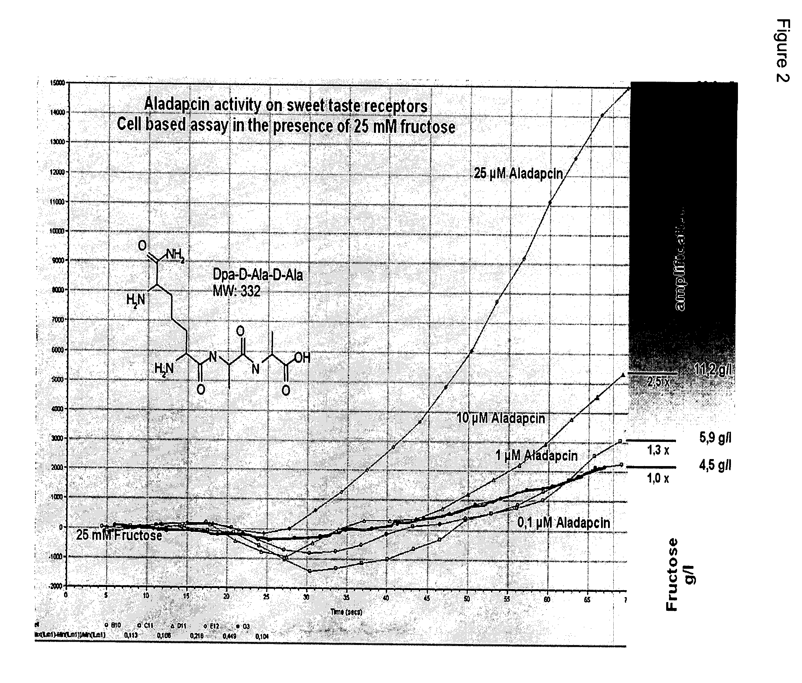

For measurement of human T1 R2/T1 R3 taste receptor dependent activity HEK293 cells stably expressing G-alpha-15, human T1 R2 and human T1 R3 were 4x10⁴ seeded in 96-well plates and labelled with the calcium sensitive fluorescence dye Fluo4-AM (2 µM) in DMEM culture medium for one hour at 37 °C. For the measurement in a fluorescence plate reader the medium was exchanged for KH-buffer and incubated for another 15 minutes at 37 °C. Fluorescence measurement of the labelled cells was conducted in a Flex Station II fluorescence plate reader (Molecular Devices, Sunnyvale, California). Response to different concentrations of aladapcin in the presence of 25 mM fructose was recorded as Fluo4-AM fluorescence increase initiated through the T1R2/T1R3 dependent increase of the second messenger calcium. The applied fructose concentration was choosen from the results of preexaminations showing that 25 mM fructose (4.5 g/I) is a concentration which is barely activating the sweet taste receptors within this cell based assay (see Fig. 2). Thus a sweetness enhancing property of a test compound is detectable in the presence of the sweetener fructose. After obtaining calcium signals for each sample, calcium mobilization in response to tastants was quantified as the relative change (peak fluorescence F1 - baseline fluorescence F0 level, denoted as dF) from its own baseline fluorescence level (denoted as F0). Though rel. RFU is dF/F0. Peak fluorescence intensity occurred about 20-30 sec after addition of tastants. The data shown were obtained from at least two independent experiments and done in triplicates. The fructose enhancing capacity of aladapcin is depicted in Figure 2 as primary fluorescence increase curves and fructose enhancement is given in g/I fructose increase faciliated by the applied aladapcin concentrations.

### Legends

- Fig. 1: shows the multicistronic eucaryotic expression vector pTrix-Eb-R2R3. The expression of the human taste receptor genes T1 R2, T1 R3 and the blasticidin S deaminase (bsd) gene are under the control of the human elongation factor 1 alpha promoter (P-ef1a). To confer multicistronic expression on the translational level two internal ribosomal entry sites (cite-I and cite-II) have been inserted. The multicistronic unit is terminated by a simian virus 40 polyadenylation site (polyA) and depicted as "cistron" with a solid black arrow. The prokaryotic origin of replication (ori) and the kanamycin resistance gene (kan) serve for the propagation, amplification and selection of the plasmid vector in *E. coli.*
- Fig. 2: shows the aladapcin activity on sweet taste receptors (activity as sweet enhancer) in the described cell based assay in the presence of 25 mM fructose. This is depicted as primary fluorescence increase (y-axis) over time (sec / x-axis). The amplification of fructose is dose dependent and the corresponding hypothetical fructose concentration is indicated in g/I fructose.

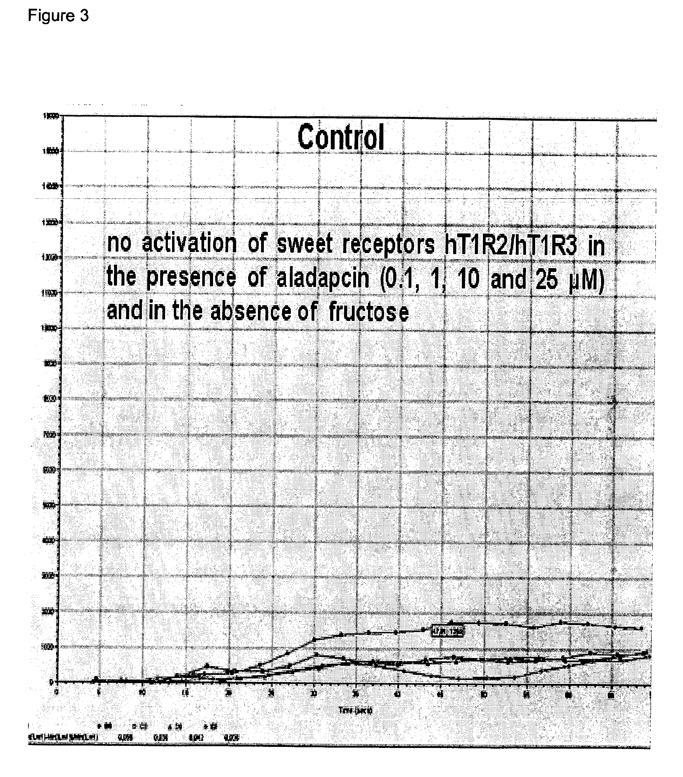

- Fig. 3: shows the results in the same test model without the presence of fructose which serve as a control measurement. The results reveal that aladapcin has no sweetener potential, at least not in the range from 0.1 - 25 µm.

## Claims

1. The use of an oligopeptide according to formula (I) 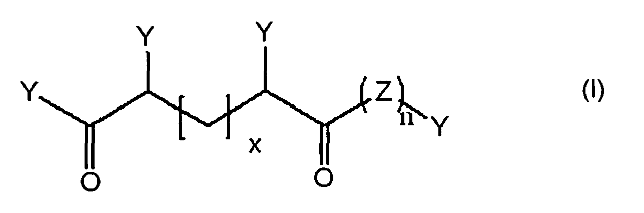 wherein
Z denotes the peptidic condensation product of alanine, arginine, asparagine, aspartic acid, cysteine, glutamic acid, glutamine, histidine, isoleucine, leucine, lysine, methionine, phenylalanine, proline, serine, threonine, tryptophan, tyrosine or valine wherein any of these amino acids is a β-D- or β-L-amino acid and wherein for n being >1 the Zs are identical or different,
n is an integer from 1 to 5
x is an integer from 1 to 5 and
Y denotes -H, -OH, -NH₂, -O-Alkyl, -O-(CO)-Alkyl, -NH-Alkyl, -N(Alkyl)₂, -NH-(CO)-Alkyl or -N((CO)-Alkyl)₂ wherein "Alkyl" is a linear or branched C₁ to C₅ alkyl rest and wherein two or more Ys are identical or different, as a taste modulator for comestible compositions.

2. The use of claim 1 wherein in formula (I) the amino acids Z are D-amino acids, specifically β-D-amino acids.

3. The use of claim 1 or 2 wherein in formula (I) Z is β-D- alanine and preferably n is 2 and x is 3 and Y is NH₂ or OH.

4. The use of one of claims 1 to 3, wherein the oligopeptide is of the formula (II), the generic name of which is Aladapcin (IUPAC: 2-[2-[(2,6-diamino-6-carbamoyl-hexanoyl)-amino]propanoylamino]propanoic acid)

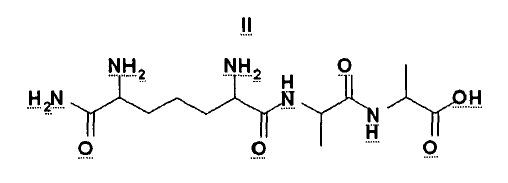

5. The use of one of claims 1 to 4, wherein the oligopeptide is used as a sweetness enhancer.

6. The use of one of claims 1 to 5, wherein the comestible composition contains at least one natural or artificial sweetener.

7. The use of one of claims 1 to 6, wherein the oligopeptide is water soluble.

8. The use of one of claims 1 to 7, wherein more than one oligopeptide is used.

9. The use of any of claims 1 to 8, wherein the oligopeptide or the oligopeptides is/are used in an amount between 0.01 mg and 10 g oligopeptide(s) / kg of the comestible composition.

10. The use of one of claims 1 to 9, wherein the comestible composition contains mono-, di- or oligosaccharides as sweeteners.

11. The use of one of claims 1 to 10, wherein the comestible composition contains high fructose corn syrup (HFCS) as sweetener.

12. The use of one of claims 1 to 11, wherein the comestible composition is selected from the group consisting of ice cream, beverages, yoghurts, desserts, spreads and medicinal compositions, preferably carbohydrated alcoholic and non-alcoholic beverages.

13. A method for the modulation of taste of a comestible composition comprising the step of adding an oligopeptide according to formula (I) as defined in claim1 to said composition.

14. A method for reducing the concentration of caloric sweeteners in a comestible composition comprising the step of adding an oligopeptide according to formula (I) as defined in claim1 to said composition.

15. A comestible composition containing an oligopeptide according to formula (I) as defined in claim1.
